(19) Europäisches Patentamt
European Patent Office
Office européen des brevets

(11) Veröffentlichungsnummer: **0 251 050**
**B1**

(12) **EUROPÄISCHE PATENTSCHRIFT**

(45) Veröffentlichungstag der Patentschrift: **23.01.91**

(51) Int. Cl.[5]: **C 07 C 49/713,**
C 07 C 49/743, C 07 C 69/145,
A 61 K 7/46, C 11 B 9/00

(21) Anmeldenummer: **87108758.1**

(22) Anmeldetag: **07.03.84**

(60) Veröffentlichungsnummer der früheren Anmeldung nach Art. 76 EPÜ: **0 119 546**

# (54) Neue 2,4,4-Trimethyl-cyclohex-2-en-1-on-derivate und deren Verwendung als Riechstoffe.

(30) Priorität: **15.03.83 DE 3309169**

(43) Veröffentlichungstag der Anmeldung:
**07.01.88 Patentblatt 88/01**

(45) Bekanntmachung des Hinweises auf die Patenterteilung:
**23.01.91 Patentblatt 91/04**

(84) Benannte Vertragsstaaten:
**CH DE FR GB LI**

(56) Entgegenhaltungen:
**EP-A-0 007 582**
**EP-A-0 012 246**

(73) Patentinhaber: **BASF Aktiengesellschaft**
**Carl-Bosch-Strasse 38**
**D-6700 Ludwigshafen (DE)**

(72) Erfinder: **Janitschke, Lothar, Dr.**
**Wormser Gasse 9**
**D-6711 Kleinniedesheim (DE)**
Erfinder: **Hoffmann, Werner, Dr.**
**Ringstrasse 11 c**
**D-6708 Neuhofen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 251 050 B1

Courier Press, Leamington Spa, England.

**Beschreibung**

Gegenstand der Erfindung sind 2,4,4-Trimethyl-cyclohex-2-en-1-on-derivate der allgemeinen Formel I

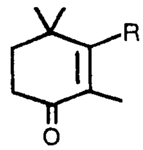

(I),

in der R für einen der folgenden Reste steht:

$—CH(OH)—CH_3$ (Ic)

$—CH_2(OH)$ (Id)

$—CH(OH)—CH{=}CH_2$ (Ie)

$—CH(OCOCH_3)—CH_3$ (If)

und

$—CH_2—OCOCH_3$ (Ig)

Die Verbindungen Ic bis Ig sind interessante Riech- und Aromastoffe. Weiterhin können sie als Zwischenprodukte für neue Carotinoide Bedeutung gewinnen. Sie können auf relativ einfache Weise aus recht gut zugänglichen Verbindungen hergestellt werden.

Ausgangsverbindungen für die herstellung der neuen Verbindungen Ic, Id und Ie sind die entsprechenden neuen Oxime der allgemeinen Formel II

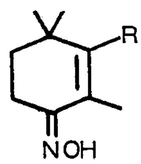

(II),

in der R

$—CH(OH)—CH_3$ (IIc)

$—CH_2OH$ (IId)

oder

$—CH(OH)—CH{=}CH_2$ (IIe)

steht,

die ihrerseits durch Grignardreaktion mit der entsprechenden Alkenyl- oder Alkylgrignardverbindung bzw. im Fall von IId durch Meerwein-Ponndorf-Verley-Reduktion mit Aluminiumisopropylat und Isobutanol aus 4-Oximido-cyclocitral der Formel III

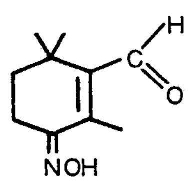

(III),

in ausgezeichneten Ausbeuten erhalten werden können.

Da III gemäß dem Verfahren der nicht vorveröffentlichten Patentanmeldung (P 31 37 802.1) auf überraschend einfache Weise und in hoher Ausbeute aus Cyclocitral hegestellt werden kann, und dieses gemäß der DE—OS 30 27 689 sehr vorteilhaft aus Citral zugänglich ist, ergibt sich somit ein sehr vorteilhaftes Gesamtverfharen zur Herstellung der neuen Riech- und Aromastoffe der Formel Ic, Id und Ie.

So können die neuen Oxime Ic, IId und IIe in an sich bekannter Weise mit wäßrig enthanolischer Natriumhydrogensulfitlösung in guter Ausbeute in die neuen Verbindungen der Formel Ic, Id und Ie überführt werden (vgl. z.B. Houben-Weyl "Methoden der organischen Chemie", Bd. 10/IV, S. 268ff).

Die Alkohole Ic und Id lassen sich dann in an sich bekannter Weise, beispielsweise durch Umsetzen mit Acetanhydrid und Pyridin in sehr guten Ausbeuten in die neuen Acetate If und Ie überführen. Bezüglich weiterer Einzelheiten über solche Veresterungen verweisen wir beispielsweise auf Fieser und Fieser "Reagents for Organic Synthesis", Vol. 1, S. 958.

Die Verbindungen Ic bis Ig sind interessante Riech- und Aromatstoffe. Weiterhin können sie als Zwischenprodukte für neue Carotinoide Bedeutung gewinnen. Sie können auf relativ einfache Weise aus recht gut zugänglichen Verbindungen hergestellt werden.

Beispiel 1

A. Herstellung von 2,4,4-Trimethyl-3-(1-hydroxy-prop-2-en-1-yl)-cyclohex-2-en-1-on-oxim (IIe)

Bei 20 bis 25°C wurde eine Lösung von 63,4 g (0,35 mol) 2,6,6-Trimethyl-3-oximido-cyclohex-1-en-1-yl-carboxaldehyd (III) in 300 ml Tetrahydrofuran (THF) zu einer Lösung von 1,5 mol Vinylmagnesiumchlorid in 1 000 ml THF getropft. Nach beendeter Zugabe wurde 1 Stunde (h) bei Raumtemperatur (RT) nachgerührt. Man kühlte die Reaktionsmischung ab und tropfte unterhalb von 10°C 150 ml Wasser zu. Anschließend wurde 15 Min. nachgerührt.

Die ausgefallenen Magnesiumsalze wurde abfiltriert und der Filterrückstand noch dreimal mit Chloroform gewaschen. Die vereinigten Filtrate wurden bei ca. 20 mbar eingeengt.

Man erhielt 83 g eines verunreinigten Rohproduktes, das aus Essigsäureethylester (Essigester) umkristallisiert wurde. Hierbei erhielt man 61 g (Ausbeute: 83,3% d. Th.) des spektrokopisch reinen 2,4,4-Trimethyl-3-(1-hydroxy-prop-2-en-1-yl)-cyclohex-2-en-1-on-oxims. Die eingeengte Mutterlauge (ca. 20 g) enthielt weitres verunreinigtes Produkt.

Fp: 138—142°C.

B. Herstellung von 2,4,4-Trimethyl-3-(1-hydroxy-prop-2-en-1-yl)-cyclohex-2-en-1-on (Ie)

36,2 g (0,17 mol) des gemäß A erhaltenen 2,4,4-Trimethyl-3-(1-hydroxy-prop-2-en-1-yl)-cyclohex-2-en-1-on-oxims (IIe) wurden zusammen mit 250 ml einer 40 %igen wäßrigen $NaHSO_3$-Lösung und 250 ml Ethanol für 2,5 h unter Rückfluß zum Sieden erhitzt.

Anschließend wurde das Ethanol bei ca. 20 mbar abdestilliert, der Rückstand mit 750 ml 10 %iger $NaHCO_3$-Lösung alkalisch gestellt und fünfmal mit Chloroform extrahiert. Die vereinigten Extrakte wurden mit Natriumsulfat getrocknet und das Lösungsmittel bei ca. 20 mbar abdestilliert.

Man erhielt 14,3 g Ie (Ausbeute: 42,6% d. Th.). Das Rohprodukt wurde aus 43 ml Cyclohexan umkristallisiert. Man erhielt hierbei 13,5 g reines Ie (Ausgeute: 40,2% d. Th.).

Fp: 93—96°C.

IR (KBr-Preßling): 3 364 $cm^{-1}$, 1 649 $cm^{-1}$, 1 605 $cm^{-1}$.

$^1H$-NMR ($CDCl_3$/80 MHz) δ = 5,83—6,25 ppm, m, 1H, CH-2'; 5,13—5,42 ppm, m, 2H, $CH_2$-3'; 5,07 ppm, m, 1H, CH-1'; 2,48 ppm, t (J ~ 7Hz), 2H, $CH_2$—6; 2,12 ppm, m, 1H, OH; 1,87 ppm, S, 3H, $CH_3$ an C-2; 1,81 ppm, t (J ~ 7Hz), 2H, $CH_2$-5, 1,28 und 1,17 ppm, je 1S, 3H, $CH_3$ an C-4.

$^{13}C$—NMR ($CDCl_3$/90,52 MHz) δ: 199,88 ppm, C-1; 161,73 ppm, C-3; 137,98 ppm, C-2'; 132,89 ppm, C-2; 115,91 ppm, C-3'; 71,62 ppm, C-1'; 37,79 ppm C-5; 36,17 ppm, C-4; 34,20 ppm, C-6; 27,25 und 26,13 ppm, $CH_3$ an C-4; 12,44 ppm, $CH_3$ an C-2.

Geruch: süßlich, fruchtig (waldmeisterartig).

(Ionon-Typ ohne Holzcharakter).

Beispiel 2

A. Herstellung von 2,4,4-Trimethyl-3-(1-hydroxy-ethyl)-cyclohex-2-en-1-on-oxim (IIc)

Analog Beispiel 1A wurden 63,4 g (0,35 mol) 2,6,6-Trimethyl-3-oximido-cyclohex-1-en-1-yl-carboxy-aldehyd (III) in 300 ml THf mit einer Lösung von 1,5 mol Methylmagnesiumchlorid umgesetzt.

Durch Aufarbeitung analog Beispiel 1A wurden 70 g eines Rohproduktes erhalten der aus Essigester umkristallisiert wurde. Hierbei erhielt man 60 g (Ausbeute: 86,9% d. Th.) spektroskopisch reines 2,4,4-Trimethyl-3-(1-hydroxy-ethyl)-cycloex-2-en-1-on-oxim. Die eingeengte Mutterlauge (ca. 10 g) enthielt weiteres Produkt.

Fp: 131—135°C.

B. Herstellung von 2,4,4-Trimethyl-3-(1-hydroxy-ethyl)-cyclohex-2-en-1-on (Ic)

Analog Beispiel 1B wurden 39,4 g (0,2 mol) 2,4,4-Trimethyl-3-(1-hydroxyethyl)-cyclohex-2-en-1-on-oxim (IIc) mit 250 ml einer 40 %igen $NaHSO_3$-Lösung und 250 ml Ethanol 2,5 h unter Rückfluß zum Sieden erhitzt.

Durch Aufarbeitung analog Beispiel $^1B$ erhielt man 34,1 g rohes Ic (Ausbeute: 93,5 d. Th.). Das Rohprodukt wurde aus 105 ml Cyclohexan umkristallisiert. Man erhielt 24,5 g des spektroskopisch reinen kristallinen Produktes (Ausbeute: 67,2% d. Th.).

Fp: 91—94°C.

IR (KBr-Preßling): 3 370 $cm^{-1}$, 1 635 $cm^{-1}$, 1 595 $cm^{-1}$.

$^1H$-NMR ($CDCl_3$/80 MHz) δ: 4,72 ppm, d, q (J1 ~ 3Hz, J2 ~ 7Hz), 1H, CH-1'; 2,45 ppm, t (J ~ 7Hz), 2H, $CH_2$-6; 2,07 ppm, d (J ~ 3Hz), 1H, OH; 1,95 ppm, S, 3H, $CH_3$ an C-2; 1,83 ppm, t (J ~ 7Hz), 2H, $CH_2$-5; 1,48 ppm, d (J ~ 7Hz), 3H, $CH_3$-2'; 1,28 und 1,16 ppm, je 1S, 3H, $CH_3$ an -C-4.

$^{13}C$-NMR ($CDCl_3$/90,52 MHz) δ: 199,96 ppm, C-1; 164,73 ppm, C-3; 131,54 ppm, C-2; 67,40 ppm, C-1'; 37,90 ppm, C-5; 35,89 ppm, C-4; 34,18 ppm, C-6; 27,43 und 25,95 ppm, $CH_3$ an -CH-4; 22,26 ppm, C-2'; 12,37 ppm, $CH_3$ an C-2.

Geruch: süßlich, fruchtig (himbeerartig, Waldmeister).

Beispiel 3

A. Herstellung von 2,4,4-Trimethyl-3-hydroxymethyl-cyclohex-2-en-1-on-oxim (IId)

90,5 g (0,5 mol) 2,6,6-Trimethyl-3-oximido-cyclohex-1-en-1-yl-carboxaldehyd (III) wurden zusammen mit 500 ml Isopropanol und 102 g (0,5 mol) Aluminium-tri-isopropylat zum Rückfluß erhitzt. Innerhalb einer Stunde wurden über eine 30 cm $V_2A$-Netz-Kolonne unterhalb von 80°C, 60 ml zunächst Aceton und später Aceton/Isopropanol-Gemische abdestilliert. Anschließend wurden weitere 300 ml Isopropanol destillativ entfernt.

Der Rückstand wurde langsam auf ein Gemisch von 1,5 kg Eis und 85 g konz. $H_2SO_4$ gegeben. Es wurde fünfmal mit Chloroform extrahiert. Die Extrakte wurden mit gesättigter $NaHCO_3$-Lösung und Wasser neutral gewaschen, mit Natriumsulfat getrocknet und das Lösungsmittel bei ca. 20 mbar abdestilliert.

Man erhielt 105 g rohes 2,4,4-Trimethyl-3-hydroxymethyl-cyclohex-2-en-1-on-oxim. Das Rohprodukt wurde aus 500 ml Essigester umkristallisiert. Hierbei wurden 91 g (Ausbeute: 99,3% d. Th.) der reinen kristallinen Verbindung erhalten. Fp: 152—155°C.

Geruch: süßlich, fruchtig (himbeerartig, Waldmeister).

B. Herstellung von 2,4,4-Trimethyl-3-hydroxymethyl-cyclohex-2-en-1-on (Id)

Analog Beispiel 1B wurden 165 g (0,9 mol) 2,4,4-Trimethyl-3-hydroxymethylcyclohex-2-en-1-on-oxim (IId) mit 1040 ml 40 %iger $NaHSO_3$-Lösung und 1040 ml Ethanol für 2,5 h unter Rückfluß zum Sieden erhitzt.

Durch Aufarbeitung analog 1B erhielt man 151,1 g rohes Id (Ausbeute: 99,8% d.Th.). Das Produkt wurde durch fraktionierte Destillation über eine 20 cm $V_2A$-Netz-Kolonne gereinigt:

Fraktion 1: Kp (0,02—0,01 mbar) 88—103°C; 10 g, GC: 81,2%

Fraktion 2: Kp (0,01 mbar) 103°C; 22,1 g, GC: 98,2%

Fraktion 3: Kp (0,01 mbar) 103°C; 103,5 g, GC: 100%.

Die Fraktionen 1 bis 3 entsprechen einer Gesamtausbeute von 88,1% d. Th. ber. auf 100 %iges Produkt.

IR (KBr-Preßling): 3 376 $cm^{-1}$, 1 647 $cm^{-1}$, 1 615 $cm^{-1}$.

$^1$N-NMR ($CDCl_3$/200 MHz) δ: 3,33 ppm, S, 2H, $CH_2$—OH; 2,50 ppm, t (J ~ 7Hz), 2H, $CH_2$-6; 1,87 ppm, S, 3H, $CH_3$ an C-2; 1,83 ppm, t (J ~ 7Hz), 2H, $CH_2$-5; 1,73 ppm, S, 1H, OH; 1,20 ppm, S, 6H, $CH_3$ an C-4.

$^{13}$C-NMR ($CDCl_3$/90,52 MHz) δ: 199,92 ppm, C-1; 160,40 ppm, C-3; 133,08 ppm, C-2; 59,47 ppm, $CH_2OH$; 37,41 ppm, C-5; 35,43 ppm, C-4; 34,33 ppm, C-6; 26,62 ppm, $2CH_3$ an C-4; 11,31 ppm, $CH_3$ an C-2.

Geruch: fruchtig, blumig (etwas fettig).

Beispiel 4

Herstellung von 2,4,4-Trimethyl-3-(1-acetoxy-ethyl)-cyclohex-2-en-1-on (If)

114 g (0,63 mol) 2,4,4-Trimethyl-3-(1-hydroxy-ethyl)-cyclohex-2-en-1-on (Ic) wurden zusammen mit 59,7 g Pyridin und 64,3 g Acetanhydrid 3 h unter Rückfluß zum Sieden erhitzt.

Anschließend wurde abgekühlt und auf 1,3 kg Eiswasser gegossen. Man extrahierte fünfmal mit Diethylether, wusch die vereinigten Extrakte mehrmals mit Wasser sowie mit ges. $NaHCO_3$-Lösung, trocknete mit Natriumsulfat und destillierte das Lösungsmittel bei ca. 20 mbar ab.

Man erhielt 148,3 g rohes If das durch fraktionierte Destillation über eine Brücke gereinigt wurde.

Fraktion 1: Kp (0,2—0,15 mbar): 96°C; 3,6 g nach GC: 95,4 %ig

Fraktion 2: Kp (0,02 mbar): 91—93°C; 13,4 g nach GC: 96,5 %ig

Fraktion 3: Kp (0,02 mbar): 92—93°C; 93,1 g nach GC: 95,8 %ig

Fraktion 4: Kp (0,02 mbar): 93°C; 29,3 g nach GC: 87,2 %ig

Rückstand 1,8 g.

Die Fraktionen 1 bis 4 ergeben zusammen, umgerechnet auf 100 %iges Produkt eine Ausbeute von 92,8% d. Th.

IR (Kbr-Preßling): 1 745 $cm^{-1}$, 1 675 $cm^{-1}$, 1 615 $cm^{-1}$.

$^1$H-NMR ($CDCl_3$/80 MHz) δ: 5,67 ppm, q (J ~ 7Hz), 1H, CH-1'; 2,47 ppm, t (J ~ 7Hz), 2H, $CH_2$-6; 2,05 ppm, S, 3H, Acetat-$CH_3$; 1,92 ppm, S, 3H, $CH_3$ an C-2; 1,79 ppm, t (J ~ 7Hz), 2H, $CH_2$-5; 1,51 ppm, d (J ~ 7Hz), 3H, $CH_3$-2'; 1,29 und 1,18 ppm, je 1S, 3H, $CH_3$ an C-4.

$^{13}$C-NMR($CDCl_3$/90,52 MHz) δ: 199,14 ppm, C-1; 169,54 ppm, Carbonyl-C der Acetylgruppe; 160,48 ppm, C-3; 132,41 ppm, C-2; 68,81 ppm, C-1'; 38,05 ppm, C-5; 35,95 ppm, C-4; 34,10 ppm, C-6; 27,76 und 25,90 ppm, $CH_3$ an C-4; 21,01 ppm, Acetat-$CH_3$; 20,00 ppm, $CH_3$-2'; 12,51 ppm, $CH_3$ an C-2.

Geruch: honigartig, suß, blumig (krautiger, Nebengeruch).

Beispiel 5

Herstellung von 2,4,4-Trimethyl-3-acetoxymethyl-cyclohex-2-en-1-on (Ig)

47 g (0,28 mol) 2,4,4-Trimethyl-3-hydroxymethyl-cyclohex-2-en-1-on (Id) wurden zusammen mit 26,54 g Pyridin und 28,6 g Acetanhydrid 3 h unter Rückfluß zum Sieden erhitzt. Nach dem Abkühlen goß man auf 200 ml Eiswasser und extrahierte fünfmal mit Ether. Die vereinigten Extrakte wurden mehrmals mit ges. $NaHCO_3$-Lösung gewaschen, mit Natriumsulfat getrocknet und das Lösungsmittel bei ca. 20 mbar abdestilliert.

Man erhielt 53,1 g rohes Ig (Ausbeute 90,3% d. Th.), die durch fraktionierte Destillation an einer 20 cm Vigreux-Kolonne gereinigt wurden.

Fraktion 1: Kp (0,03 mbar) 58—84°C; 3,4 g n. GC: 90,7 %ig Fraktion 2: Kp (0,01 mbar) 85—100°C; 5,9 g n.

GC: 93,6 %ig Fraktion 3: Kp (0,01 mbar) 99°C; 36,5 g n. GC: 99,9 %ig Fraktion 4: Kp (0,01 mbar) 99°C; 2,2 g n. GC 100,0 %ig.

Die Fraktionen 1 bis 4 entsprechen einer Ausbeute von 80,9% d.Th., berechnet auf 100 %iges Produkt.

IR (Film): 1 727 $cm^{-1}$, 1 665 $cm^{-1}$, 1 607 $cm^{-1}$.

$^1$H-NMR ($CDCl_3$/80 MHz) δ: 4,65 ppm, S, 2H, $CH_2$-OAc; 2,46 ppm, t (J ≡ 7Hz), 2H, $CH_2$-6; 2,04 ppm, S, 3H, Actyl-$CH_3$; 1,81 ppm, t (J ~ 7Hz), 2H, $CH_2$-5; 1,78 ppm, S, 3H, $CH_3$ an C-2; 1,17 ppm, S, 6H, $CH_3$ an C-4.

$^{13}$C-NMR ($CDCl_3$/90,52 MHz) δ: 199,61 ppm, C-1; 170,42 ppm, Acetyl-C=O; 155,02 ppm, C-3; 135,12 ppm, C-2; 60,98 ppm, $CH_2$ an C-3; 37,44 ppm, C-5; 35,50 ppm, C-4; 34,29 ppm, C-6; 26,54 ppm, $CH_3$ an C-4; 20,70 ppm, Acetyl-$CH_3$; 11,44 ppm, $CH_3$ an C-2.

Geruch: süß, fruchtig, holzig.

## Patentansprüche

1. 2,4,4-Trimethyl-cyclohex-2-en-1-on-derivate der allgemeinen Formel I

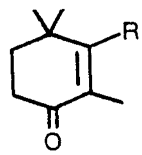 (I),

in der R für einen der folgenden Reste steht:

$-CH(OH)-CH_3$ (Ic)

$-CH_2(OH)$ (Id)

$-CH(OH)-CH=CH_2$ (Ie)

$-CH(OCOCH_3)-CH_3$. (If)

$-CH_2-OCOCH_3$ (Ig)

2. 2,4,4-Trimethyl-3-(1-hydroxy-prop-2-en-yl)-cyclohex-2-en-1-on (Ie).
3. 2,4,4-Trimethyl-3-(1-hydroxyethyl)-cyclohex-2-en-1-on (Ic).
4. 2,4,4-Trimethyl-3-hydroxymethyl-cyclohex-2-en-1-on (Id).
5. 2,4,4-Trimethyl-3-1-acetoxyethyl)-cyclohex-2-en-1-on (If).
6. 2,4,4-Trimethyl-3-acetoxymethyl-cyclohex-2-en-1-on (Ig).
7. Verwendung der Verbindungen der allgemeinen Formel I gemäß Anspruch 1 als Riechstoffe.

## Revendications

1. Dérivés de 2,4,4-triméthyl-cyclohex-2-ène-1-one de formule générale

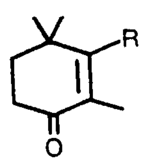 (I),

dans laquelle R est mis pour un des restes suivantes:

$-CH(OH)-CH_3$ (Ic)

$-CH_2(OH)$ (Id)

$-CH(OH)-CH=CH_2$ (Ie)

$-CH(OCOCH_3)-CH_3$ (If)

$-CH_2-OCOCH_3$ (Ig)

2. 2,4,4-Triméthyl-3-(1-hydroxy-prop-2-ène-1-yl)-cyclohex-2-ène-1-one (Ie).
3. 2,4,4-Triméthyl-3-(1-hydroxyéthyl)-cyclohex-2-ène-1-one (Ic).
4. 2,4,4-Triméthyl-3-(1-hydroxyméthyl-cyclohex-2-ène-1-one (Id).
5. 2,4,4-Triméthyl-3-(1-acétoxyéthyl)-cyclohex-2-ène-1-one (If).

6. 2,4,4-Triméthyl-3-(1-acétoxyméthyl-cyclohex-2-ène-1-one (Ig).
7. Utilisation des composés de formule générale I selon la revendication 1 comme parfums.

**Claims**

1. A 2,4,4-trimethylcyclohex-2-en-1-one derivative of the formula I

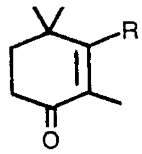

(I),

where R is one of the following radicals:

$$—CH(OH)—CH_3 \quad (Ic)$$

$$—CH_2(OH) \quad (Id)$$

$$—CH(OH)—CH=CH_2 \quad (Ie)$$

$$—CH(OCOCH_3)—CH_3 \quad (If)$$

$$—CH_2—OCOCH_3 \quad (Ig)$$

2. 2,4,4-Trimethyl-3-(1-hydroxyprop-2-en-1-yl)-cyclohex-2-en-1-one (Ie).
3. 2,4,4-Trimethyl-3-(1-hydroxyethyl)-cyclohex-2-en-1-one (Ic).
4. 2,4,4-Trimethyl-3-(1-hydroxymethyl-cyclohex-2-en-1-one (Id).
5. 2,4,4-Trimethyl-3-(1-acetoxyethyl)-cyclohex-2-en-1-one (If).
6. 2,4,4-Trimethyl-3-acetoxymethyl-cyclohex-2-en-1-one (Ig).
7. Use of a compound of the formula I as claimed in claim 1 as a scent.